# EUROPEAN PATENT APPLICATION

(11) **EP 2 612 646 A1**
(43) Date of publication of application: **10.07.2013**
(21) Application number: 12171825.8
(22) Date of filing: 13.06.2012
(51) Int. Cl.: A61K 8/04, A61K 8/34, A61K 8/81, A61Q 17/00, A01N 25/04, A61K 31/045

(54) **Process for the production of alcoholic gels for skin and hand disinfection**

(30) Priority: 11.07.2011 DE 102011078966
(71) Applicant: L'AIR LIQUIDE, SOCIETE ANONYME POUR L'ETUDE ET L'EXPLOITATION DES PROCEDES GEORGES CLAUDE, 75007 Paris (FR); SCHÜLKE & MAYR GMBH, 22851 Norderstedt (DE)
(72) Inventor: Steinhauer, Katrin, 22459 Hamburg (DE); Kolditz, Petra, 22145 Hamburg (DE); Da Silva Nolasco, Angelo, 22111 Hamburg (DE)
(74) Representative: Conan, Philippe Claude

(57) **Abstract**

The invention relates to a process for the production of an alcoholic gel which comprises
a) at least 20% by weight of aliphatic alcohol and
b) at least one thickener,
in which
1) a phase I is provided, where at least one thickener based on acrylic acid polymer is dispersed in a liquid initial charge, where the liquid initial charge comprises one or more aliphatic C₃- to C₈-alcohols,
2) a phase II is provided which comprises water and/or one or more aliphatic alcohols,
3) phase I and phase II are mixed together in order to produce a mixture, and
4) at least one neutralizing agent is added to the mixture.

Surprisingly, during the production process according to the invention and also upon use of the alcoholic gels according to the invention, lump formation in the gels does not arise.

## Description

The present invention relates to a process for the production of alcoholic gels for skin and hand disinfection, and to a gel which can be produced according to the process.

Alcoholic gels for skin and hand disinfection, and also processes for their production are known. The content of alcohol ensures their good disinfecting effect. The content of thickeners in turn ensures that the disinfectant amount of alcohol required for a reliable disinfection effect is spread uniformly over the hands and remains on the skin for the period required for a good disinfection effect.

However, it has been found that the industrial production of alcoholic gels by known processes leads to lumps. These lumps settle out in the production vessels and can only be removed from them with very great difficulty. Consequently, the vessels have to be laboriously cleaned and are therefore sometimes not available for up to several days. These problems during production occur for example with thickeners based on acrylic acid polymers and are also observed during the use of such alcoholic gels.

Thickeners based on acrylic acid polymers (also referred to as carboxyvinyl polymers, generic name: carbomer) are sold, for example, by The Lubrizol Corporation under the trade name Carbopol®. The gel formation of aqueously dispersed acrylic acid polymers starts after adding a base. As a result, the carboxyl groups are deprotonated and repel one another: the acrylic acid polymer swells up and forms a colourless gel.

US 4,956,170 A describes an alcoholic gel composition for disinfecting the hands. The thickener used is an addition polymer of acrylic acid (crosslinked with unsaturated polyfunctional agents). To produce the gel composition of US 4,956,170, the addition polymer is dispersed in alcohol and then fatty acid ester humectant is incorporated into the dispersion. Any further constituents which may be present (including further thickeners) are dispersed separately in water. The two dispersions are then combined. Finally, a neutralizing agent of the amino alcohol type is added in order to effect the gelation.

EP 0 604 848 A2 already describes that the use of alcoholic gels containing thickeners based on acrylic acid polymers leads to the formation of residues (resin formation) of the polymer component. To solve this problem, a high molecular weight cellulose compound is used together with the acrylic acid polymer. The acrylic acid polymer is homogenized in water and gelated with neutralizing agent. An alcoholic solution of the cellulose is added to this gel. However, cellulose thickeners leave behind a sticky feel on the skin. Also, no process for the production of the gels is given in EP 0 604 848 A2.

EP 1 964 576 A1 teaches that a disinfecting gel preparation should generally comprise healing, alleviating, care and/or anti-inflammatory active ingredients. To produce the gel preparation, the alcohol component is mixed with the required amount of water, then the active ingredient components that are readily soluble in alcoholically aqueous media are added, if appropriate a solubilizer is added and then the other components are added. After adding the thickener, the mixture is homogenized and, if necessary, the pH is adjusted.

WO 03/003998 A1 describes a process for the production of disinfecting skin and hand care gels. In this,
1) an acrylic acid polymer is added to an initial charge of water and the polymer is dispersed until a milky-cloudy, homogeneous dispersion is formed,
2) an alcoholic phase is added to the dispersion,
3) the polymer solution formed is neutralized with at least one neutralizing agent and
4) at least 5% by weight, based on the total amount of the gel, of at least one alcohol of the general formula R-OH, in which R is a aliphatic, linear or branched hydrocarbon radical having 3 to 8 carbon atoms, is added to the overall mixture.

Optionally, step 3) and 4) according to WO 03/003998 A can take place simultaneously, i.e. the neutralizing agent is mixed with the C₃- to C₈- alcohol.

Upon adding acrylic acid polymer in H₂O as per EP 1 964 576 A1 or WO 03/003998 A1, small gel lumps are immediately formed, which can only be dissolved again with difficulty. Furthermore, these lumps firmly adhere to the stirrer and to the vessel wall and can only be removed again by means of a high mechanical cleaning expenditure.

Accordingly, the object of the present invention is to provide a process which limits, and preferably excludes, the problems described above. In particular, the aim was to avoid, by virtue of the process, the formation of lumps during production and use.

Surprisingly, it has now been found that these problems are solved by a process for the production of an alcoholic gel which comprises
a) at least 20% by weight of aliphatic alcohol and
b) at least one thickener,
in which
1) a phase I is provided, where at least one thickener based on acrylic acid polymer is dispersed in a liquid initial charge, where the liquid initial charge comprises one or more aliphatic C₃- to C₈-alcohols,
2) a phase II is provided which comprises water and/or one or more aliphatic alcohols,
3) phase I and phase II are mixed together in order to produce a mixture, and
4) at least one neutralizing agent is added to the mixture.

Contrary to the teaching of WO 03/003998 A1, it is thus surprisingly not necessary to add C₃- to C₈-alcohol after adding the neutralizing agent (in order to effect the swelling and gelation of the acrylic acid polymer). The invention is based inter alia on the fact that it has been found that alcoholic gels produced according to the invention do not have a tendency to form lumps during production and also during subsequent use.

In the production according to the invention, the phases I and II are mixed in a step 3). These two phases and their production, and also further, optional constituents of the alcoholic gels are described in more detail below.

In the process according to the invention, the procedure in step 3) can for example involve initially introducing phase I and admixing phase II. Alternatively, it is possible to introduce phase II as initial charge and to add phase I. Thus, firstly phase I and then phase II can be produced, or firstly phase II and then phase I can be produced.

In the process according to the invention, it is also possible that in step 3) Phase I is introduced as initial charge and then, if phase II consists of two or more constituents, these two or more constituents of phase II are added individually to phase 1. However, in all embodiments of the invention, it is important that in phase I the thickener based on acrylic acid polymer is dispersed in the liquid initial charge with a content of C₃- to C₈-alcohol.

### Production of phase I

In the production of phase I, at least one thickener based on acrylic acid polymer is dispersed in a liquid initial charge. The liquid initial charge comprises one or more aliphatic C₃- to C₈-alcohols.

Exemplary acrylic acid polymers are disclosed in the prior art cited above. Preferably, the acrylic acid polymer used according to the invention is a crosslinked acrylate/C₁₀- to C₃₀-alkyl acrylate polymer, which is optionally hydrophobically modified. Examples of preferred acrylic acid polymers are Carbopol® ETD 2020, Carbopol® Ultrez 20 and Carbopol® 981 NF, which are all obtainable from The Lubrizol Corporation.

Preferred alcohols of the liquid initial charge for phase I are selected from 1-propanol, 2-propanol (isopropyl alcohol, isopropanol), 1-butanol, isobutyl alcohol, tert-butyl alcohol, 1-pentanol, 2-pentanol, 3-pentanol and neopentyl glycol alcohol, where 1-propanol, 2-propanol and mixtures thereof are preferred, and 2-propanol is particularly preferred, and where phase II is particularly preferably a mixture of water with ethanol.

It is thus preferred that the aliphatic C₃- to C₈-alcohol of the liquid initial charge is 2-propanol.

It is also preferred that the amount of C₃-to C₈-alcohol is such that, based on the total mass of the finished gel, it is 1 to 20% by weight, preferably 2 to 15% by weight, in particular 3 to 10% by weight, such as, for example, about 3% by weight or about 10% by weight.

Moreover, it is preferred that phase I comprises at most 30% by weight of water, preferably at most 20% by weight, more preferably at most 10% by weight, in particular at most 5% by weight, such as at most 3% by weight, at most 2% by weight, or at most 1% by weight, in each case based on the total mass of phase I, where a phase I which is essentially water-free is particularly preferred. Essentially water-free means that phase I comprises no added water and thus only comprises the negligible amount of water which usually accompanies the use of the constituents of phase I.

### Production of phase II

The production process according to the invention includes the provision of a phase II which comprises water and/or one or more aliphatic alcohols.

Preferred aliphatic alcohols of phase II are selected from ethanol, 1-propanol, 2-propanol, 1-butanol, isobutyl alcohol, sec-butyl alcohol, tert-butyl alcohol, 1-pentanol, 2-pentanol, 3-pentanol and neopentyl alcohol, where ethanol is particularly preferred.

Here, the fraction of aliphatic alcohol in phase II is preferably 50 to 98% by weight, such as, for example, 65 to 96% by weight, more preferably 70 to 94% by weight, such as 75 to 92% by weight, based on the total mass of phase II.

Moreover, preference is given to a phase II which comprises a mixture of water with one or more aliphatic alcohols, where a mixture of water with ethanol is particularly preferred.

Exemplary neutralizing agents used in stage 4) of the process according to the invention for the gelation of the acrylic acid polymer are likewise disclosed in the prior art cited above. Preferred neutralizing agents are triethanolamine, tromethamine (tris(hydroxymethyl)-aminomethane), PEG-15-cocamine, isopropylamine, diisopropanolamine, triisopropanolamine and aminomethylpropanol. The neutralizing agent is particularly preferably N,N,N',N'-tetrakis(2-hydroxypropyl)ethylenediamine, which is available as Lutropur® Q75 or Neutrol® TE from BASF SE.

Besides the constituents of aliphatic alcohol and acrylic acid polymer, alcoholic gels produced according to the invention can comprise one or more from spreading agents, refatting agents, further thickeners, perfumes, colorants and biocides, which are mixed into the gel. These further constituents can be added to the liquid initial charge for phase I, phase I, phase II, to the mixture of phase I and/or later.

Preferably, spreading agent, refatting agent and/or further thickener are mixed in during the production of phase I, where spreading agent and refatting agent are particularly preferably present in the liquid phase into which the acrylic acid polymer is then incorporated. Preferred further thickeners are vinyl polymers such as carboxyvinyl polymers, cellulose ethers such as hydroxypropylmethylcellulose, hydroxyethylcellulose, carboxymethylcellulose, guar gum and anthan gum.

Another group of constituents of the alcoholic gels produced according to the invention, namely one or more from further refatting agents, perfumes, colorants and biocides, are preferably mixed in stage 4). These specified agents can be mixed in after adding the neutralizing agent in order to then produce the end product. However, it is preferred that these agents are mixed in before the addition of the neutralizing agent in order to then produce the end product.

### Alcoholic gels produced according to the invention

Gels according to the invention preferably comprise 30% by weight of water, based on the total mass of the gel, for example 5 to 25% by weight of water.

The alcoholic gel comprises at least 20% by weight of aliphatic alcohol. This includes the amount of aliphatic alcohol (preferably ethanol) introduced with phase II and the amount of aliphatic C₃- to C₈-alcohol (preferably 2-propanol) introduced via phase I. Preferred amounts of aliphatic alcohol in the gel are at least 30% by weight of aliphatic alcohol, preferably at least 40% by weight of aliphatic alcohol, more preferably at least 50% by weight of aliphatic alcohol, in particular at least 60% by weight of aliphatic alcohol, such as 65 to 96% by weight of aliphatic alcohol, preferably 70 to 93% by weight of aliphatic alcohol, in particular 74 to 91% by weight of aliphatic alcohol (in each case based on the total mass of the gel).

In addition, the invention also relates to an alcoholic gel which can be produced, and is preferably produced, by the process according to the invention.

Particular preference is given to the following formulations X and Y for the alcoholic gel produced according to the invention (quantitative data in % by weight):

**Formulation X for an alcoholic gel**

| **Alcoholic gel X** | **Preferably** | **Particularly preferably** | **Especially** |
|---|---|---|---|
| Ethanol | 60-95 | 65-93 | 70-88 |
| Acrylic acid polymer | 0.1-1.0 | 0.2-0.6 | 0.3-0.5 |
| Isopropyl alcohol | 4-20 | 6-15 | 8-12 |
| Neutralizing agent | 0.1-1.5 | 0.3-1.1 | 0.5-0.9 |
| Refatting system | 0.4-3.0 | 0.7-2.0 | 1.0-1.6 |
| Further refatting agent | 0.02-1.0 | 0.05-0.6 | 0.1-0.3 |
| Biocide (2-biphenylol) | 0.01-1.0 | 0.02-0.3 | 0.05-0.2 |
| Water | ad 100 | ad 100 | ad 100 |

**Formulation Y for an alcoholic gel**

| **Alcoholic gel Y** | **Preferably** | **Particularly preferably** | **Especially** |
|---|---|---|---|
| Ethanol | 55-91 | 60-86 | 65-81 |
| Acrylic acid polymer | 0.01-1.0 | 0.02-0.5 | 0.05-0.2 |
| Isopropyl alcohol | 0.5-15 | 1-10 | 2-5 |
| Neutralizing agent | 0.01-1.0 | 0.02-0.5 | 0.05-0.2 |
| Refatting system | 0.4-3.0 | 0.7-2.0 | 1.0-1.6 |
| Further refatting agent | 0.02-1.0 | 0.05-0.6 | 0.1-0.3 |
| Biocide (2-biphenylol) | 0.02-2.0 | 0.05-0.6 | 0.1-0.3 |
| Water | ad 100 | ad 100 | ad 100 |

The process according to the invention is associated with the following advantages:
- reduction in cleaning expenditure due to improved and more homogeneous production of alcoholic gels,
- possibility of reducing the production costs as a result of lower cleaning expenditure and
- more rapid reusability of the production containers used and also consequently reduction in production time.

The advantages of the invention arise in particular from the examples below. Unless stated otherwise, the percentages are by weight (% by weight).

### Examples

Two alcoholic gels were produced using the constituents listed below, once by the process according to the invention, and another time by the process as per WO 03/003998 A1.

| No. | Constituent | Amount [% by wt.] |
|---|---|---|
| 1 | Isopropyl alcohol | 3.00 |
| 2 | Refatting system | 1.30 |
| 3 | Acrylic acid polymer | 0.100 |
| 4 | Ethanol | 73.0 |
| 5 | Water | ad 100 |
| 6 | Neutralizing agent | 0.100 |
| 7 | Further refatting agent | 0.200 |
| 8 | Biocide (2-biphenylol) | 0.100 |

### Example 1 (according to the invention)

Firstly, a phase I was produced by mixing the constituents Nos. 1 and 2 together. A clear solution was formed. Acrylic acid polymer (3) was then added. The solution became white/cloudy in colour. The mixture was then stirred for 30 minutes and then left to swell overnight with slow stirring. The solution formed was cloudy.

A Phase II was then produced by mixing ethanol and water (4 and 5). phase I was transferred to phase II. After adding neutralizing agent (6), the solution thickened and became clear. The addition of further refatting agent (7) and biocide (8) led to a weakly opal coloured, clear alcoholic gel with a viscosity of 331.5 mPa*s.

### Example 2 (comparison)

In a comparative experiment, the constituents 1 to 8 given above were formulated as described in WO 03/003998 A in order to produce a gel with a viscosity of 354.79 mPa*s. Upon adding acrylic acid polymer in H₂O, small gel lumps were immediately formed, which could only be dissolved again with difficulty. Furthermore, these lumps adhered firmly to the stirrer and to the vessel wall and could only be removed again by means of a high mechanical cleaning expenditure.

## Claims

1. Process for the production of an alcoholic gel which comprises
a) at least 20% by weight of aliphatic alcohol and
b) at least one thickener,
in which
1) a phase I is provided, where at least one thickener based on acrylic acid polymer is dispersed in a liquid initial charge, where the liquid initial charge comprises one or more aliphatic C₃- to C₈-alcohols,
2) a phase II is provided which comprises water and/or one or more aliphatic alcohols,
3) phase I and phase II are mixed together in order to produce a mixture, and
4) at least one neutralizing agent is added to the mixture.

2. Process according to claim 1, **characterized in that** the one or more aliphatic C₃- to C₈-alcohols in the liquid initial charge for phase I is or are selected from 1-propanol, 2-propanol, 1-butanol, isobutyl alcohol, tert-butyl alcohol, 1-pentanol, 2-pentanol, 3-pentanol and neopentyl alcohol, where 1-propanol, 2-propanol and mixtures thereof are preferred, and 2-propanol is particularly preferred.

3. Process according to claim 1 or 2, **characterized in that** the amount of C₃- to C₈-alcohol is 1 to 20% by weight, based on the total mass of the finished gel, preferably 2 to 15% by weight.

4. Process according to one of the preceding claims, **characterized in that** phase I comprises at most 30% by weight of water, preferably at most 20% by weight, more preferably at most 10% by weight, in particular at most 5% by weight, such as at most 3% by weight, at most 2% by weight, or at most 1% by weight, in each case based on the total mass of phase I, where a phase I which is essentially water-free is particularly preferred.

5. Process according to one of the preceding claims, **characterized in that** the one or more aliphatic alcohols of phase II is or are selected from ethanol, 1-propanol, 2-propanol, 1-butanol, isobutyl alcohol, sec-butyl alcohol, tert-butyl alcohol, 1-pentanol, 2-pentanol, 3-pentanol and neopentyl alcohol, where ethanol is particularly preferred.

6. Process according to one of the preceding claims, **characterized in that** the fraction of aliphatic alcohol in phase II is 50 to 98% by weight, preferably 65 to 96% by weight, more preferably 70 to 94% by weight, such as 75 to 92% by weight, based on the total mass of phase II.

7. Process according to one of the preceding claims, **characterized in that** phase II comprises a mixture of water with one or more aliphatic alcohols, preferably a mixture of water with ethanol.

8. Process according to one of the preceding claims, **characterized in that** the alcoholic gel comprises at most 30% by weight of water, based on the total mass of the gel, preferably 5 to 25% by weight of water.

9. Process according to one of the preceding claims, **characterized in that** the gel comprises at least 30% by weight of aliphatic alcohol, preferably at least 40% by weight of aliphatic alcohol, more preferably at least 50% by weight of aliphatic alcohol, in particular at least 60% by weight of aliphatic alcohol, such as 65 to 96% by weight of aliphatic alcohol, preferably 70 to 93% by weight of aliphatic alcohol and in particular 74 to 91% by weight of aliphatic alcohol (in each case based on the total mass of the gel).

10. Process according to one of the preceding claims, **characterized in that** the acrylic acid polymer is a crosslinked acrylate/C₁₀- to C₃₀-alkyl acrylate polymer.

11. Process according to one of the preceding claims, **characterized in that** one or more from spreading agents, refatting agents, further thickeners, perfumes, colorants and biocides is or are mixed into the gel.

12. Process according to Claim 11, **characterized in that** spreading agent, refatting agent and/or further thickener is or are mixed in during the production of phase I, where preferably spreading agent and refatting agent are present in the liquid initial charge into which the acrylic acid polymer is incorporated.

13. Process according to Claim 11 or 12, **characterized in that** the further refatting agent, perfume, colorant and/or biocide is or are mixed in in stage 4), where the specified agents are preferably mixed in before adding the neutralizing agent.

14. Alcoholic gel which can be produced by the process according to one of the preceding claims.
